# EUROPEAN PATENT SPECIFICATION

(11) **EP 1 380 313 B1**
(45) Date of publication and mention of the grant of the patent: **18.05.2005**
(21) Application number: 03013341.7
(22) Date of filing: 16.06.2003
(51) Int. Cl.: A61L 27/46, A61K 6/033, C04B 38/06, A61L 27/56, A61L 27/12

(54) **Method of preparing porous calcium phosphate morsels and granules via Gelatin processing**
Verfahren zur Herstellung poröser Calciumphosphatstückchen und -granulaten aus der Gelatineverarbeitung
Procédé de préparation de morceaux et de granules de phosphate de calcium poreux par traitement utilisant de la gélatine

(30) Priority: 11.07.2002 EP 02015436
(43) Date of publication of application: 14.01.2004
(73) Proprietor: Biomet Deutschland GmbH, 14167 Berlin (DE)
(72) Inventor: Tas, Ahmet Cuneyt, Dr., Ankara 06370 (TR)
(74) Representative: Gross, Felix, Dr.

(56) References cited:
- BIGI A. ET AL: "Bonelike apatite growth on hydroxyapatite-gelatin sponges from simulated body fluid" J. BIOMED. MATER. RES., vol. 59, 2002, pages 709-714, XP002260940
- DATABASE WPI Section Ch, Week 198725 Derwent Publications Ltd., London, GB; Class D22, AN 1987-174387 XP002260941 & JP 62 105950 A (SUMITOMO ALUMINIUM SEIREN KK), 16 May 1987 (1987-05-16)

## Description

The invention relates to a method of preparing porous alpha- or beta-tricalcium phosphate (TCP), brushite (CaHPO₄·2H₂O), calcium pyrophosphate (Ca₂P₂O₇) or hydroxyapatite (Ca₅(PO₄)₃(OH) or mixtures thereof in the form of morsels (cylinders) or granules via Gelatin processing. These morsels or granules can be used as bone or tooth fillers or bone substitutes in applications, especially when higher rates of resorption or taking part in the bone remodelling processes of the implanted material are desired.

As the materials used for artificial bone, artificial tooth and compensation of bones (hereinafter referred to as "bone filler") in dentistry, cerebral surgery and orthopedic surgery, those non-toxic, sufficient in mechanical strength, high affinity towards a living body so as to facilitate the direct bonding therewith, and naturally in vivo so as to be naturally replaceable by a newly formed bone are preferred.

As a method for producing such a bone filler with a highly porous structure, it is known to mix a suitable raw material powder with a thermally decomposable material, molding the mixture into a pre-selected form, and performing the removal of the thermally decomposable material and sintering of the raw material powder by consecutive heating (see a.) A. Slosarczyk, "Highly Porous Hydroxyapatite Material," *Powd. Metal. Int.,* 21, 24-25 (1989), b.) L. Menabue, L. Forti, G. Pellacani, "A Study of Materials Suitable to Produce Bioceramics with Controlled Porosity for Prosthetic Implants Stabilized by Bone Tissue Ingrowth," *Biomaterials,* 6, 3-4 (1992), c.) Dean-Mo Liu, "Fabrication and Characterization of Porous Hydroxyapatite Granules," *Biomaterials,* 17, 1955-1957 (1996), d.) M. Fabbri, G.C. Celotti, and A. Ravaglioli, " Granulates Based on Calcium Phosphate with Controlled Morphology and Porosity for Medical Applications: Physico-Chemical Parameters and Production Technique," *Biomaterials.* 15, 474-477 (1994), e.) E. Ryshkewitch, "Compression Strength of Porous Sintered Alumina and Zirconia" *J. Am. Ceram. Soc.,* 157, 65-68 (1953), f.) N. Passuti, G. Daculsi, J.M. Rogez, S. Martin, and J.V. Bainvel, "Macroporous Calcium Phosphate Ceramic Performance in Human Spine Fusion," *Clin. Orth. Rel. Res.,* 248, 169-176 (1989), g.) H.S. Byrd, P.C. Hobar, and K. Shewmake, "Augmentation of the Craniofacial Skeleton with Porous HA Granules," *Plast. Reconstr. Surg.,* 91, 15-26 (1993), h.) J.F. Piecuch, R.G. Topazian, S. Skoly, and S. Wolfe, "Experimental Ridge Augmentation with Porous HA Implants," *J. Dent. Res.,* 62, 148-154 (1983), i.) Japanese Patent Laid-Open No. 60-21763, j.) Japanese Patent Laid-Open No. 60-16879, and k.) N. O. Engin and A. C. Tas, "Manufacture of Macroporous Calcium Hydroxyapatite Bioceramics," *J. Euro. Ceram. Soc.,* 19 (13-14), 2569-2572 (1999)).

In these known methods of preparing porous calcium phosphates, however, the contact of the thermally decomposable material added (typically in the form of a solid substance) for formation of pores is not necessarily uniform, and the formed pores are mostly apt to be open cells. Even if the formed adjacent pores are in contact and continued to each other, the sectional area of the communicating part of each pore is minimized. In such a pore structure, it is difficult to make cells necessary for bone formation (osteoblasts and related cells) intrude uniformly into each pore.

Natural bones do basically consist of inorganic calcium phosphate and fibrous organic collagen. Gelatin being the denatured form of collagen, has a significantly high solubility even in water at room temperature. Gelatin, depending on its concentration, may form a viscous, thermo-reversible gel with water, and its use as a biomedical polymer in surgical operations have already been documented (Y. Otani, Y. Tabata, and Y. Ikada, "Adhesion to Soft Tissues by Gelation-Polyanion Hydrogels," *J. Adhesion,* 59, 197-205 (1999)).
Y. Fujishiro et al., "Preparation and Compressive Strength of alpha-tricalcium phosphate/gelatin gel composite cement," *J. Biomed. Mater. Res.,* 54, 525-530 (2001) and A. Bigi et al. "Bonelike Apatite Growth on Hydroxyapatite-Gelatin Sponges from Simulated Body Fluid," *J. Biomed. Mater. Res.,* 59, 709-714 (2002) disclose gelatin as a pore-former in the production of porous calcium phosphate-based biomedical implants.
However, the methods described in these studies implied the use (i.e., implantation) of such calcium phosphate-gelatin composites without a total burnout/removal of the numerous organic amino-acids and other substances (which result from the dissolution/decomposition of gelatin in aqueous media in the presence of calcium phosphates).

It must be remembered that gelatin is not such a simple material to start with. Although before its hydrolysis in aqueous media it is a well-defined material, following its dissolution in water (the extent of its dissolution and the exact occurrence of its decomposition products heavily depending on the temperature of solution and its concentration), it turns into a quite complex mixture of organic acids.

The following table (from J. A. Arnesen, *et al*., *Bioresource Technology, 82,* 191-194 (2002)) compares the amino-acid compositions of different mammalian gelatins in hydrolyzed gelatin samples, whereas the numbers denote "moles per 100 mole of amino acids." Therefore, utmost caution must be exercised while the use of gelatin sponges mixed with calcium phosphates as a direct implantation material (without a thermal decomposition / burn-out step) is considered.

| Amino acid | Porcine | Bovine | Whale |
|---|---|---|---|
| Glycine | 30.8 | 33.3 | 30.2 |
| Proline | 12.7 | 12.4 | 10.8 |
| Alanine | 11.1 | 11.5 | 10.4 |
| Hydroxyproline | 10.9 | 9.6 | 8.5 |
| Glutamic acid | 7.8 | 7.4 | 8.0 |
| Arginine | 5.1 | 4.6 | 5.3 |
| Aspartic acid | 4.4 | 4.3 | 4.8 |
| Serine | 3.3 | 3.2 | 4.0 |
| Lysine | 2.7 | 2.6 | 3.0 |
| Leucine | 2.6 | 2.4 | 2.8 |
| Valine | 2.3 | 2.0 | 2.2 |
| Threonine | 1.8 | 1.7 | 2.9 |
| Phenyalanine | 1.3 | 1.3 | 1.5 |
| Isoleucine | 1.1 | 1.2 | 1.2 |
| Hydroxlysine | 0.7 | 0.7 | 0.9 |
| Methionine | 0.5 | 0.5 | 0.6 |
| Histidine | 0.4 | 0.5 | 0.6 |
| Ornithine | 0.2 | 0.6 | 0.0 |
| Tyrosine | 0.2 | 0.1 | 0.5 |

Many of these amino-acids, at the levels indicated in the above table, when incorporated in an implant material must be thoroughly tested for the presence of any adverse or side effects on the implant site. In other words, the presence of such organic acids may readily alter the expected bone-healing behavior of the calcium phosphates used together with them.

An object of the present invention is to provide a method for preparing porous alpha- or beta-TCP, brushite (CaHPO₄·2H₂O), calcium pyrophosphate (Ca₂P₂O₇) or hydroxyapatite (Ca₅(PO₄)₃(OH)) or mixtures thereof in the form of morsels or granules via gelatin processing, which avoids the above-mentioned disadvantages from the prior art.
Upon further study of the specification and appended claims, further objects and advantages of this invention will become apparent to those skilled in the art.

These objects are achieved by a method of preparing porous alpha- or beta-TCP, brushite (CaHPO₄·2H₂O), calcium pyrophosphate (Ca₂P₂O₇) or hydroxyapatite (Ca₅(PO₄)₃(OH)) or mixtures thereof in the form of morsels or granules via gelatin processing characterized in that the method comprises the steps of:
a) mixing a calcium phosphate self-setting cement powder and gelatin powder in a ratio of 3 : 0.25 to 1.
b) adding Na₂HPO₄ solution followed by mixing the formed paste.
c) placing the formed paste into a syringe immediately.
d) squeezing out the morsels of the syringe after a few minutes.
e) placing the morsels, after removing it out of the syringe, directly in distilled water at 37 °C for a few days to dissolve away gelatin and to form interconnected pores.
f) Thermal treatment to burnout all organic or volatile material followed by successive cooling to room temperature.
g) Optionally crushing of the calcined, sintered morsels and then sieving to obtain porous granules.

### Detailed description of the invention

According to the invention the following starting materials (calcium phosphates) are preferred:
a) Chemically synthesized α-TCP powder (where TCP is Ca₃(PO₄)₂ with a Ca/P molar ratio of 1.50)
b) Chemically synthesized β-TCP powder
c) Chemically synthesized "bi-phasic" mixtures of HA and α-TCP powder (where HA is Ca₅(PO₄)₃(OH) with a Ca/P molar ratio in the range of 1.51 to 1.65
d) Chemically synthesized DCPD powder (dicalcium phosphate dihydrate, CaHPO₄ ·2 H₂O with Ca/P = 1.00)
e) DCPA powder (dicalcium phosphate anhydrous, CaHPO₄ with Ca/P=1.00 obtained by heating DCPD powders (of item d above) at 120°C)
f) Chemically synthesized ACP powder (amorphous calcium phosphate, with Ca/P being variable over the range of 0.8 to 1.60)

The above-mentioned calcium phosphate powders when mixed with one another in proper ratios will result in self-setting calcium phosphate cements. Mixing ratios of the powders listed above will give the user a freedom in adjusting the Ca/P molar ratio of the final powder body. Some of those powders are not self-setting cements by themselves, but upon mixing with one another they become one. The use of self-setting calcium phosphate cement formulations, as a starting material, provides the unique ability to easily impart any desired shape to the final product, which shall not just be restricted to the shape of small cylinders/morsels.

Following table, for example, will explain the mixing order and percentages of the individual powders to yield self-setting cements:

| Powder | | Mixing ratio (weight fraction) | Ca/P ratio range to be attained |
|---|---|---|---|
| 1 | A | 1 | 1.50 |
| 2 | A+B | ¼ B+ ¾ A | 1.50 |
| 3 | C | 0.05 HA + 0.95 α-TCP | 1.54-1.55 |
| 4 | C+D | ¾ C + ¼ D | 1.39 |
| 5 | A+C+E+CaCO₃ | 0.63 + 0.02 + 0.25 + 0.10 | 1.51-1.52 |
| 6 | F + D | 0.60 to 0.80 F + 0.40 to 0.20 D | 1.00 to 1.50 |

Each one of these 6 powders given in the above table can be taken as the starting self-setting calcium phosphate cement, and then these powders are to be mixed with proper amounts of Gelatin (Merck KGaA, Gelatin powder food grade, Cat. Nr. 104078) by ball milling or by mixing with hand in an agate mortar with an agate pestle.

There are two processing possibilities from this point onwards:
1.) If the formed morsel needs further machining (i.e., cutting, slicing, drilling, etc.), the morsel must be kept dry at room temperature for around 2 days. Within 2 days the morsel reaches a compressive strength of around 20 MPa, and it can then be machined.
2.) If the morsel does not require any machining, it can be placed directly in distilled water at 37°C, for 2 days, after removing it out of the syringe.

The second route is the most preferred one, since the "soaking-in-water" step will dissolve away gelatin and it will form interconnected pores. Amino acids formed during the dissolution of gelatin component will also result in the local dissolution (in the micron-levels) of the calcium phosphate matrix, and create a communicating network of micropores around the macropores generated by the leached out gelatin particles. The sizes of the formed macropores essentially depend on the initial particle size of gelatin powder used, which was around 250 to 400 µm.

After the proper choice of one of the above-mentioned possibilities (dictated by the product specifications, i.e., slices, holes to be drilled, oblique angles on one end of the morsel, etc.), both kind of samples will receive the same thermal treatment to burnout all the organic or volatile material.

When heated alone in air, gelatin totally volatilizes at around 750°C. However, when the total burnout of gelatin is achieved at that temperature, the remaining porous skeleton of calcium phosphate does not have the required mechanical stability to be handled, and for that reason the thermal treatment temperature must be pushed upwards until the sintering temperature of the calcium phosphate compound under consideration. In the case of the material of this example, the sintering temperature for TCP is 1200°C.
Morsels dried at 37°C prior to the thermal treatment were placed into an electrically-heated chamber furnace on Al₂O₃ flat plates, and then heated from RT to 1200°C in 500 minutes, soaked at 1200°C for 360 minutes, followed by cooling to RT. At this point arised two more possibilities for the producer, if the morsels were quenched from 1200° to 1000°C in 10 minutes, the high-temperature polymorphic form of TCP can be brought down to RT, and the product will consist of single-phase α-TCP, and if the morsels were slowly cooled within the furnace (from 1200°C to RT in 6 hours), then the samples will be single-phase β-TCP. Intermediate cooling rates (i.e., between those of quenching and slow cooling, e.g., cooling from 1200° to 1000°C in 1 h) will result in the formation of bi-phasic (i.e., almost equimolar mixtures of α- and β-TCP phases) TCP materials. Since the beta form is more resorbable as compared to the alpha form, the control (in terms of thermal treatment regimes utilized) to be gained over the mixing ratio of these two phases in the final product will provide a quite useful tool in tailoring in the *in vivo* resorption rates of these morsels.

Gelatin powder is preferably mixed with calcium phosphate powders in a mixing ratio of 0.25 to 1 : 3, most preferably 0.7 to 1 : 3.
The most preferred range of total porosity to be achieved (without destroying the cylindrical morsel or any other initially intended geometrical shape) is 35 to 50 %.

Similarly, the initial particle size distribution possessed by the gelatin powder (porcine) strongly affect the sizes of the macropores to be attained in the final products. The used gelatin powder has 45% dry particles in the range of 250 to 700 µm (with the average particle size in this range being observed at around 400 µm), and the rest were smaller than 250 µm. Average of the macropores observed in the calcined products were in the range of 300 to 400 µm, whereas the average of micropores were observed in the range of 3 to 5 µm. All macropores were connected to one another with the micropores. A self-setting cement is a special (or appropriate) mixture of more than one component (except of α-TCP which is by itself a low-strength self-setting cement) of calcium phosphate compounds to be selected either from the binary system of CaO-P₂O₅ or from the ternary system of CaO-P₂O₅-H₂O, which starts to set when mixed with a small amount of pure water (and more preferably, when mixed with a small amount of water which contains small amounts (1 to 4 wt%) of a basic phosphate compound, such as Na₂HPO₄).

The morsels are preferably cylinders with diameters variable within the range of 0.5 to 2.5 cm, most preferably of 1 cm diameter, and having a height of 1 to 4 cm.

The granules are irregularly shaped particles with a granule size distribution easily adjustable (achieved by sieving) over the range of 0.5 to 5 mm.

The invention is described in detail below in terms of the following working examples.

In the foregoing and in the following examples, all temperatures are set forth uncorrected in degrees Celsius; and, unless otherwise indicated, all parts and percentages are by weight.

### Example 1

### Production of Porous Morsels of Ca₃(PO₄)₂: ("Injection molding")

30 grams of α-TCP powder (powder A) and 10 grams of Gelatin powder were mixed in a plastic bottle in a Turbula mill for 1 hour. Then, a 4.0 grams portion of this mixture was placed into an agate mortar. 2.5 mL of 3 wt% aqueous Na₂HPO₄·2H₂O solution (or 2.75 mL of 2 wt% Na₂HPO₄·2H₂O solution) was added to the mortar with a pipette, followed by mixing the formed paste with an agate pestle for 30 seconds. The formed paste (mainly with the immediate chemical reaction taking place between the basic (pH>9) setting solution and gelatin) was immediately placed into a 5 mL syringe within around 2 minutes after the mixing of the powder and setting solution described above, and the morsel was squeezed out of the syringe after 10 minutes. The procedure described here can be named as the injection molding of a viscous paste of calcium phosphate+gelatin mixtures. The mold can therefore have any geometrical shape as desired.
There are two processing possibilities from this point onwards:
1.) If the formed morsel needs further machining (i.e., cutting, slicing, drilling, etc.), the morsel must be kept dry at room temperature for around 2 days. Within 2 days the morsel reaches a compressive strength of around 20 MPa, and it can then be machined.
2.) If the morsel does not require any machining, it can be placed directly in distilled water at 37°C, for 2 days, after removing it out of the syringe.
The second route is the most preferred one.
Then, the thermal treatment to burnout all the organic or volatile compounds follows.
When heated alone in air, gelatin totally volatilizes at about 750 °C. The thermal treatment temperature must be pushed upwards until the sintering temperature (i.e. for TCP = 1200 °C).

Morsels dried at 37°C prior to the thermal treatment were placed into an electrically-heated chamber furnace on Al₂O₃ flat plates, and then heated from RT to 1200°C in 500 minutes, soaked at 1200°C for 360 minutes, followed by cooling to RT. At this point arised two more possibilities for the producer, if the morsels were quenched from 1200° to 1000°C in 10 minutes, the high-temperature polymorphic form of TCP can be brought down to RT, and the product will consist of single-phase α-TCP, and if the morsels were slowly cooled within the furnace (from 1200°C to RT in 6 hours), then the samples will be single-phase β-TCP. Intermediate cooling rates (i.e., between those of quenching and slow cooling, e.g., cooling from 1200° to 1000°C in 1 h) will result in the formation of bi-phasic TCP materials.

### Example 2

### Production of Porous Granules:

Calcined, sintered morsels are crushed, and then sieved with a series of the following sieves of respective opening sizes: 5 mm, 2.8 mm, 2 mm, 1.25 mm and 1 mm. Granules formed in this way possess irregular shapes. However, prolonged sieving has the proven tendency to round off the sharp edges of those.

### Example 3

### Evaluation of the porosity and pore size distribution in morsels or granules:

Total porosity in the produced morsels or granules are directly determined by the density measurements, based on a gas-absorption technique. The theoretical density of the calcium phosphate compounds are well known and do only slightly vary from one another over the range of 3.1 to 3.2 g/cm³. Experimentally measured densities of each sample are divided by the theoretical density of the specific phase comprising the sample was made out of, and multiplied by 100. The resultant number, when subtracted from 100 gives the total porosity in the samples. Percentage of total porosity is then reported on a statistical average basis for that batch of samples. Pore size measurements were performed by using a scanning electron microscopy (SEM) on Au-Pd coated (20-50 angstrom) samples. Macropore and micropore sizes are then directly measured from the enlarged SEM photomicrographs. Density measurements and SEM analysis were performed both on morsels and granules. Crushing of the morsels to form granules does not change the pore size distribution in the granules (as compared to the mother morsels).

### Example 4

### Control of porosity and pore size distribution in morsels and granules:

The amount of gelatin powder initially blended with the calcium phosphate powders strongly influence the total porosity in the final products. Gelatin powder was most preferably to be mixed with 3 grams of calcium phosphate powder over the range of 0.25 to 1 g. When the gelatin amount was increased beyond 1 g (up to 2 g), consecutive soaking of the formed morsels in water lead to the losing of the formed shape. The most preferred range of gelatin addition to 3 g of calcium phosphates is 0.7 to 1 g. The most preferred range of total porosity to be achieved by this technique (without destroying the cylindrical morsel or any other initially intended geometrical shape) is 35 to 50%. Interconnecting pores are dynamically formed within the first half hour during the soaking of the forms in water. The forms must not be kept in water for more than 3 hours, in order not to destroy their shapes. The undissolved portion of the gelatin is removed during the calcination/sintering step.

### Example 5

### Production of Porous Morsels with a Phase Mixture of Ca₂P₂O₇ and Ca₃(PO₄)₂:

22.47 grams of powder C (a bi-phasic (95%-5%) mixture of α-TCP and calcium hydroxyapatite, HA), 7.53 g powder D (CaHPO₄·2H₂O) and 10 grams of Gelatin powder were mixed in a plastic bottle in a Turbula mill for 1 hour. Then, a 4.0 grams portion of this mixture was placed into an agate mortar. 2.5 mL of 3 wt% aqueous Na₂HPO₄·2H₂O solution (or 2.75 mL of 2 wt% Na₂HPO₄·2H₂O solution) was added to the mortar with a pipette, followed by mixing the formed paste with an agate pestle for 30 seconds. The formed paste (mainly with the immediate chemical reaction taking place between the basic (pH>9) setting solution and gelatin) was immediately placed into a 5 mL syringe within around 2 minutes after the mixing of the powder and setting solution described above, and the morsel was squeezed out of the syringe after 10 minutes. Formed morsel was soaked in distilled water at 37°C for 2 days, followed by drying at 60°C overnight. The morsels produced in this way were then heated in an air atmosphere to 1250°C in 500 min, kept at that temperature for 6 hours, and then cooled to RT within the electrically-heated chamber furnace in 6 hours. X-ray diffraction analysis performed on the samples indicated the presence of 30 to 35% Ca₂P₂O₇ and 65 to 70% β-TCP. Owing to the initial Ca/P molar ratio of 1.39 utilized in the starting powder mix, these porous morsels are expected to show better resorption characteristics as compared to those made out of only TCP. The granules of this material were easily prepared by crushing and sieving the above.

## Claims

1. A method of preparing porous alpha- or beta-tricalcium phosphate, brushite (CaHPO₄·2H₂O), calcium pyrophosphate (Ca₂P₂O₇) or hydroxyapatite (Ca₅(PO₄)₃(OH)) or mixtures thereof in the form of morsels or granules **characterized in that** the method comprises the steps of:
a) mixing a calcium phosphate self-setting cement powder and gelatin powder in a ratio of 3 : 0.25 to 1.
b) adding a Na₂HPO₄ solution followed by mixing the formed paste.
c) placing the formed paste into a syringe immediately.
d) squeezing out the morsels of the syringe after a few minutes.
e) placing the morsels, after removing it out of the syringe, directly in distilled water at 37 °C for a few days to dissolve away gelatin and to form interconnected pores.
f) thermal treatment to burnout all organic or volatile material followed by successive cooling to room temperature.
g) optionally crushing of the calcined, sintered morsels and then sieving to obtain porous granules.

2. A method according to claim 1 **characterized in that** the method comprising the steps of:
a) to g) according to claim 1 and additionally after step d)
d₁) keeping dry the formed morsels at room temperature for about 2 days for further machining.

3. The method according to either of the preceding claims **characterized in that** the mixing ratio according to step a) is 3 : 0.7 to 1.

4. The method according to any of claims 1 to 3 **characterized in that** according to step f) the thermal treatment temperature is pushed upwards until the sintering temperature of the respective calcium phosphate compound.

## Patentansprüche

1. Verfahren zur Herstellung von porösem alpha- oder beta-Tricalciumphosphat, Brushit (CaHPO₄·2H₂O), Calciumpyrophosphat (Ca₂P₂O₇) oder Hydroxylapatit (Ca₅(PO₄)₃(OH)) oder Gemischen davon in Form von Teilchen oder Granulat, **dadurch gekennzeichnet, daß** man:
a) selbstabbindenden Calciumphosphatzement in Pulverform und Gelatinepulver in einem Verhältnis von 3:0,25 bis 1 vermischt;
b) eine Na₂HPO₄-Lösung zusetzt und die gebildete Paste vermischt;
c) die gebildete Paste sofort in eine Spritze einbringt;
d) nach einigen Minuten die Teilchen aus der Spritze drückt;
e) die Teilchen nach der Austragung aus der Spritze direkt einige Tage in destilliertes Wasser bei 37°C einbringt, wodurch Gelatine herausgelöst wird und interkonnektierende7 Poren gebildet werden;
f) eine Wärmebehandlung zum Ausbrennen aller organischen oder flüchtigen Substanzen durchführt und danach sukzessive auf Raumtemperatur abkühlt;
g) gegebenenfalls die calcinierten, gesinterten Teilchen zerkleinert und dann siebt, wobei man poröses Granulat erhält.

2. Verfahren nach Anspruch 1, **dadurch gekennzeichnet, daß** man:
a) bis g) gemäß Anspruch 1 durchführt und zusätzlich nach Schritt d)
d₁) die gebildeten Teilchen zur weiteren maschinellen Bearbeitung etwa 2 Tage bei Raumtemperatur hält.

3. Verfahren nach einem der vorhergehenden Ansprüche, **dadurch gekennzeichnet, daß** sich das Mischungsverhältnis gemäß Schritt a) auf 3:0,7 bis 1 beläuft.

4. Verfahren nach einem der Ansprüche 1 bis 3, **dadurch gekennzeichnet, daß** man gemäß Schritt f) die Wärmebehandlungstemperatur bis zur Sintertemperatur der jeweiligen Calciumphosphatverbindung erhöht.

## Revendications

1. Procédé pour préparer de l'alpha-phosphate tricalcique ou du bêta-phosphate tricalcique, de la brushite (CaHPO₄.2H₂O), du pyrophosphate de calcium (Ca₂P₂O₇) ou de l'hydroxyapatite (Ca₅(PO₄)₃(OH)) poreux ou des mélanges de ceux-ci sous forme de morceaux ou de granules, **caractérisé en ce que** le procédé comprend les étapes:
a) de mélange d'une poudre de ciment autodurcissant de phosphate de calcium et de poudre de gélatine dans un rapport de 3 : 0,25 à 1.
b) d'addition d'une solution de Na₂HPO₄ suivie d'un mélange de la pâte formée.
c) de placement immédiat de la pâte formée dans une seringue.
d) d'expulsion des morceaux de la seringue après quelques minutes.
e) de placement des morceaux, après les avoir enlevés de la seringue, directement dans de l'eau distillée à 37°C pendant quelques jours pour dissoudre la gélatine et pour former des pores interconnectés.
f) de traitement thermique pour calciner tous les matériaux organiques ou volatils, suivi d'un refroidissement consécutif à température ambiante.
g) éventuellement de broyage des morceaux calcinés, frittés puis tamisage pour obtenir des granules poreux.

2. Procédé selon la revendication 1, **caractérisé en ce que** le procédé comprend les étapes de
a) à g) selon la revendication 1 et en outre, après l'étape d)
d₁) maintien au sec des morceaux formés à température ambiante pendant environ 2 jours pour un usinage supplémentaire.

3. Procédé selon l'une quelconque des revendications précédentes, **caractérisé en ce que** le rapport de mélange selon l'étape a) est de 3 : 0,7 à 1.

4. Procédé selon l'une quelconque des revendications 1 à 3, **caractérisé en ce que**, selon l'étape f), la température de traitement thermique est augmentée jusqu'à la température de frittage du composé de phosphate de calcium formé.
